(19) Europäisches Patentamt
European Patent Office
Office européen des brevets

(11) **EP 3 106 201 B1**

(12) **EUROPÄISCHE PATENTSCHRIFT**

(45) Veröffentlichungstag und Bekanntmachung des Hinweises auf die Patenterteilung:
**21.11.2018  Patentblatt 2018/47**

(51) Int Cl.:
*A61N 1/04* *(2006.01)*      *A61N 1/08* *(2006.01)*
*A61N 1/18* *(2006.01)*      *A61N 1/05* *(2006.01)*

(21) Anmeldenummer: **16170561.1**

(22) Anmeldetag: **20.05.2016**

(54) **ELEKTRODENFIXIERHÜLSE MIT HAFTVERMITTELNDER OBERFLÄCHENSTRUKTUR**

ELECTRODE FIXATING SLEEVE HAVING AN ADHESION-ENHANCING SURFACE STRUCTURE

MANCHON DE FIXATION D'ELECTRODES COMPRENANT UNE STRUCTURE DE SURFACE ADHESIVE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.06.2015   DE 102015108670**
**02.06.2015   DE 102015108672**
**02.06.2015   DE 102015108671**

(43) Veröffentlichungstag der Anmeldung:
**21.12.2016   Patentblatt 2016/51**

(73) Patentinhaber: **BIOTRONIK SE & Co. KG**
**12359 Berlin (DE)**

(72) Erfinder:
• **Kolberg, Gernot**
**12161 Berlin (DE)**
• **Friedrich, Michael**
**14532 Kleinmachnow (DE)**

(74) Vertreter: **Galander, Marcus**
**Biotronik SE & Co. KG**
**Corporate Intellectual Properties**
**Woermannkehre 1**
**12359 Berlin (DE)**

(56) Entgegenhaltungen:
WO-A1-2010/083554      DE-B3-102005 015 487
US-A1- 2003 100 937      US-A1- 2005 004 640
US-A1- 2008 009 928      US-A1- 2014 276 407

## Beschreibung

Die Erfindung betrifft eine Elektrodenfixierhülse mit haftvermittelnder Oberflächenstruktur. Ferner ist ein System, bestehend aus der Elektrodenfixierhülse und einer Elektrodenleitung Gegenstand der vorliegenden Erfindung.

Herz-Kreislauf-Erkrankungen zählen zu den schwerwiegendsten Krankheiten der modernen Gesellschaft. Viele Erkrankungen verlaufen auch heute noch tödlich. Vor allem ältere Menschen sind von Herz-Kreislauf-Erkrankungen betroffen. Angesichts der steigenden Lebenserwartung und der wachsenden Anzahl chronischer Herzerkrankungen ist deshalb mit einer weiteren Zunahme dieser Krankheiten zu rechnen. Immer wieder angeführte Hauptursachen sind verbreitete Einflussfaktoren einer modernen und global vernetzten Gesellschaft wie Stress, Rauchen, zu fettes Essen, damit einhergehendes Übergewicht oder Bluthochdruck. Aber auch genetische Disposition oder Virusinfektionen können Herzerkrankungen verursachen. Da sich Störungen des Herz-Kreislauf-Systems sich bereits in jüngeren Jahren manifestieren und besonders jüngere Menschen von Beginn an in einer diese Einflussfaktoren begünstigenden Umwelt aufwachsen, ist mit einer weiteren Verstärkung des Trends zu Herz-Kreislauf-Erkrankungen zu rechnen. Einen wichtigen Ansatzpunkt stellt deshalb die konsequente Verbesserung der medizinischen Versorgung dar.

Dies führt zum einen zu steigenden Kosten und zum anderen auch zu höheren Anforderungen an die Sicherheit medizintechnischer Produkte, zum Beispiel bei Herzschrittmachern, da zukünftig tendenziell eine zunehmende Anzahl dieser Systeme im Umlauf sein wird und die Sicherheit gegen Störungen entsprechend groß sein muss.

Durch eine inhomogene Kontraktion der einzelnen Areale der linken Herzkammer kann eine Herzschwäche, auch Herzinsuffizienz genannt, entstehen. Hauptgrund für die inhomogene, beziehungsweise asynchrone Kontraktion ist eine Störung im Reizleitungssystem des Herzens. Durch die sogenannte Cardiac Resynchronization Therapy, kurz CRT-Stimulation, wird die Kontraktion der linken Herzkammer wieder homogen, vereinfacht ausgedrückt resynchronisiert, und die Pumpkraft des Herzens kann sich wieder erholen. Das Verfahren sieht die Verwendung von zwei Elektroden vor. Eine Elektrode wird in die rechte Herzkammer implantiert. Das Zielgefäß für die, die linke Herzkammer betreffende, Elektrode ist der Coronarsinus. So wird das Gefäß bezeichnet, welches das venöse Blut aus den Herzkranzgefäßen in den rechten Vorhof leitet. Die Elektrode wird in einen Seitenast des Coronarsinus implantiert und liegt auf der Außenseite der linken Herzkammer. Durch die simultane Impulsabgabe über beide Elektroden wird der linke Teil des Herzens wieder synchron elektrisch erregt und eine homogene Kontraktion ist möglich.

Die Befestigung der elektrischen Leitungen zu den Elektroden erfolgt über sogenannte Elektrodenfixierhülsen (EFH). Darunter versteht man eine spezielle Fixierhülse, die an einer definierten Stelle fest auf der Elektrodenleitung angeordnet wird und die dann an geeigneter Stelle im menschlichen Körper befestigt wird, zum Beispiel durch Annähen im Bereich eines Muskels oder eines Gefäßes. Bei neueren Varianten werden spezielle Fixierhülsen verwendet, die zusammengepresst oder festgeschraubt werden und dann eine feste Einheit mit der Elektrode bilden. Häufig sind solche Elektrodenfixierhülsen aus einem weichen Kunststoff hergestellt, werden über die Elektrodenleitung geschoben und dann mit einem auch Ligatur genannten Nähfaden angepresst. Durch die entstehende Haftreibung wird die Elektrode axial auf der Elektrodenleitung fixiert. Die Elektrodenfixierhülse kann dann an geeigneter Stelle im Körper vernäht werden.

Die DE 82 24 292 U1 beschreibt eine implantierbare Leitung mit mindestens einem elektrischen Leiter, der durch eine körperverträgliche Ummantelung isoliert ist und an dessen distalen Ende eine Elektrode befestigt ist. An dem proximalen Ende des Leiters ist eine Leitungs-Befestigungseinrichtung vorgesehen, die als eine die Ummantelung umgreifende und auf der Ummantelung verschiebbar gelagerte Verankerungshülse ausgeführt ist. Die Verankerungshülse weist eine umlaufende Nut auf, mit der die Verankerungshülse mit einem Faden vernäht werden kann.

Damit eine konventionelle Elektrodenfixierhülse mit der notwendigen axialen Haltekraft auf einer Elektrodenleitung fixiert wird, muss der Ligaturfaden sehr stark angezogen werden. Durch die dabei auf die Elektrodenleitung wirkende Radialkraft, kann die Elektrodenleitung in Mitleidenschaft gezogen werden. So wird das Isolationsmaterial, beziehungsweise die Ummantelung gequetscht und der Elektrodeninnenaufbau komprimiert. Im ungünstigsten Fall kann das zu einem Elektrodenbruch im Bereich der Elektrodenfixierhülse führen.

Das Deutsche Patent DE 10 2005 015 487 B3 Beschreibt eine Vorrichtung zum Fixieren einer Elektrode, insbesondere einer Herzschrittmacherelektrode, im Schrittmacherbett und vor dem Eintritt der Elektrode in eine Vene (Ligaturschutz) mit einer in Längsrichtung vorzugsweise über ihre gesamte Länge geschlitzten Hülse, die eine in axialer Richtung durchgehende Innenhöhlung zur Aufnahme der Elektrode hat.

Die Aufgabe der vorliegenden Erfindung besteht darin, eine Elektrodenfixierhülse der eingangs genannten Gattung zu schaffen, durch deren Verwendung in einem System mit einer Elektrodenleitung selbst dann keine negative Beeinflussung der Elektrodenleitung stattfindet, wenn der Elektrodenfixierhülse eine sehr große im Wesentlichen radiale Kraft aufgeprägt wird.

Die eingangs gestellte Aufgabe wird durch einen Gegenstand mit den Merkmalen des Patentanspruchs 1 gelöst. Offenbart wird zudem eine technische Lehre zur Gestaltung eines Systems nach Anspruch 9, dessen Elemente die Elektrodenfixierhülse und eine Elektrodenleitung sind.

Vorgesehen ist eine Elektrodenfixierhülse, wobei erfindungsgemäß eine innere Mantelfläche der Elektrodenfixierhülse zumindest teilweise mit einer haftvermittelnden Oberflächenstruktur versehen ist. Da die haftvermittelnde Oberflächenstruktur selbst über haftvermittelnde Eigenschaften verfügt, bietet dies den Vorteil, dass eine Befestigung der Elektrodenfixierhülse erfolgen kann, ohne eine große radiale Kraft auf eine Elektrodenleitung auszuüben. Eine bekannte Ursache für Beschädigungen der Elektrodenleitung wird dadurch vorteilhaft eliminiert.

Die Elektrodenfixierhülse ist derart ausgebildet, dass sich die haftvermittelnde Oberflächenstruktur nahe genug an der Oberflächenstruktur der Elektrodenleitung befindet, wenn die Elektrodenfixierhülse auf der Elektrodenleitung angeordnet ist. Bevorzugt ist ein Durchmesser der inneren Mantelfläche der Elektrodenfixierhülse, auf der die haftvermittelnde Struktur aufgebracht ist, so ausgelegt, dass sich, wenn die Elektrodenfixierhülse konzentrisch mit der Elektrodenleitung angeordnet ist, ein radiales Spaltmaß zwischen der inneren Mantelfläche der Elektrodenfixierhülse und einer äußeren Oberfläche der Elektrodenleitung einstellt, welches innerhalb eines zulässigen Bereichs liegt. Die innere Mantelfläche entspricht im Sinne der vorliegenden Erfindung zumindest abschnittsweise einer innen liegenden Oberfläche eines Hohlzylinders. Weiterhin weist die Elektrodenleitung des beschriebenen Systems bevorzugt eine rotationssymmetrische Gestalt auf, insbesondere mit einem kreisförmigen Querschnitt. Hieraus ergibt sich, dass die Auslegung des Durchmessers der inneren Mantelfläche der Elektrodenfixierhülse systemabhängig ist und von einem Außendurchmesser der Elektrodenleitung abhängt. Somit ist die Angabe einer parametrischen Auslegungsvorschrift für den Durchmesser der inneren Mantelfläche der Elektrodenfixierhülse sinnvoll, da der Fachmann den Gegenstand der vorliegenden Erfindung sonst nicht ausführen könnte. Die Auslegungsvorschrift lautet:

$$D = d + 2 \cdot R$$

wobei D den Durchmesser der inneren Mantelfläche der Elektrodenfixierhülse bezeichnet, d einen frei wählbaren Parameter bezeichnet, der dem Außendurchmesser der Elektrodenleitung entspricht und R dem radialen Spaltmaß entspricht. Der zulässige Wertebereich für das radiale Spaltmaß R beträgt zwischen 10 $\mu$m und 1500 $\mu$m, weiterhin bevorzugt zwischen 20 $\mu$m und 750 $\mu$m und besonders bevorzugt zwischen 50 $\mu$m und 300 $\mu$m. Das radiale Spaltmaß R und der frei wählbare Parameter d sind systemabhängige Größen. Mit anderen Worten: R stellt sich erst durch eine Formation des Systems, bestehend aus der Elektrodenfixierhülse und der Elektrodenleitung ein, wenn d und D vorab festgelegt werden, oder weiterhin mit anderen Worten, eine vorteilhafte Auslegung des Durchmessers D ergibt sich aus der Auslegungsvorschrift, wenn d frei gewählt wird und für R ein bevorzugter Wert eingesetzt wird.

Vorzugsweise ist die haftvermittelnde Struktur als eine Geckostruktur ausgebildet. Die Erfindung nutzt somit eine alternative Möglichkeit der Verbindung unterschiedlicher Oberflächen über das Phänomen der Trockenadhäsivität. Unter Trockenadhäsivität wird vorliegend die Ausbildung von adhäsiven Kräften zwischen Oberflächen ohne haftvermittelnde Stoffe, wie Klebstoffe, verstanden. Solche Haftsysteme sind beispielsweise auch aus der Natur, z. B. bei Gecko- oder Insekten-Beinen, bekannt. Man nimmt an, dass bei solchen Systemen die Haftkräfte auf van-der-Waals-Kräften basieren. Die hafterzeugende Oberfläche weist dazu eine haftvermittelnde Oberflächenstruktur auf, beispielsweise eine Vielzahl von borsten- oder haarförmigen Elementen, die zu einer sehr starken Vergrößerung der zur Verfügung stehenden Kontaktfläche führen. Mit der Vergrößerung der Kontaktfläche nimmt in der Folge die Stärke der bei der Kontaktierung ausgebildeten Adhäsionskräfte zu. Der Einsatz derartiger haftvermittelnder Oberflächenstrukturen wird beispielsweise von Alborz Mahdavi et al., ,A biodegradable and biocompatible gecko-inspired tissue adhesive', PNAS (2008), Vol. 105, No. 7, 2307-231, vorgeschlagen. Bei der Trockenadhäsivität hängt demnach die Stärke der Adhäsion zwischen zwei Oberflächen mit der für die Adhäsion verfügbaren Fläche zusammen. Zwei gleichförmige Oberflächen, zum Beispiel eine innere und eine äußere zylindermantelförmige Fläche oder zwei planare Flächen, haften deutlich besser aneinander, als zum Beispiel eine planare und eine sphärische Oberfläche. Allgemein gilt, je grösser die zur Adhäsion verfügbare Fläche ist, desto besser haften zwei Oberflächen aneinander.

Daraus ergibt sich, dass die erfindungsgemäße Elektrodenfixierhülse auch Element in alternativen Systemen sein kann, die als weiteres Element einen alternativen Fügepartner, zum Beispiel ein Kabel, einen Schlauch oder dergleichen aufweisen. Prinzipiell sind alle Fügepartner geeignet, die eine zu der inneren Mantelfläche der Elektrodenfixierhülse passende Oberflächenform aufweisen. Wenn im Sinne der vorliegenden Erfindung die folgenden Ausführungen weiterhin am Beispiel der Elektrodenleitung erfolgen, so impliziert dies keinesfalls, dass sich diese auch auf die Elektrodenleitung beschränken.

Die haftvermittelnde Oberflächenstruktur kann beispielsweise zwischen 10 und 1.000.000 Stäbchen pro Quadratmillimeter aufweisen. Das Verhältnis von Durchmesser und Länge der Stäbchen kann zwischen 1:2 und 1:2000 liegen. Der Querschnitt des Stäbchens kann querprofiliert sein, zum Beispiel ganz oder teilweise rund, dreieckig, rechteckig, quadratisch oder innen hohl. Er kann ein T-Profil haben oder den Umrissen einer Mondsichel entsprechen. Dadurch kann eine Vorzugsbiegerichtung des Stäbchens vorgegeben werden. Alternativ oder in Kombination können die Stäbchen vorgebogen oder schräg angebracht sein. Eine einheitliche Biegerichtung der Stäbchen kann verhindern, dass die Stäbchen sich untereinander verheddern. Die Stäbchen können ferner ein Längsprofil haben. So können sie an der Wurzel, wo sie an der zu fixierenden Komponente anliegen verdickt sein und sich zum Ende hin verjüngen.

Die haftvermittelnde Oberflächenstruktur kann auch aus sich verästelnden Stäbchen bestehen. Das Ende des letzten Zweiges kann wieder verdickt sein. Die größte Ausdehnung der Verdickung entspricht maximal dem 100-fachen des Stäbchendurchmessers, auf dem die Verdickung sitzt. Das Ende des letzten Zweiges kann auch planar oder abgerundet sein oder spitz auslaufen. Am Ende des letzten Zweiges kann sich eine lappenartige Struktur, ähnlich einer Kelle, befinden, die einseitig angebunden ist. Die lappenartige Struktur ist vorzugsweise einseitig an die Stäbchen angebunden in der Art, dass der Winkel der Stäbchen fortgesetzt wird. So wird bei einer Querkraft der Komponente in die ablösende Richtung (zum Beispiel entgegen dem Uhrzeigersinn) die lappenartige Struktur vom Gewebe geschält, was das Ablösen erheblich erleichtert, während bei Querkraft in die andere Richtung nur eine Scherkraft verursacht wird, die die Fixierung nicht nur nicht ablöst, sondern unterstützt.

Durch Gestaltung der Biegerichtung der Stäbchen auf der Fläche können die Fixations- und Ablösekräfte eingestellt werden. Die Strukturen fixieren besonders gut, wenn möglichst viele Stäbchen gleichzeitig die Zugkräfte aufnehmen. Soll die Fixierung gelöst werden, müssen die Stäbchen möglichst einzeln belastet werden, um auch mit geringen Kräften eine Ablösung zu ermöglichen. Durch die Vorzugsbiegerichtung der Stäbchen kann eine seitlich auf die Komponente wirkende Kraft je nach Richtung in eine Zugkraft oder in eine Druckkraft umgesetzt werden. Eine Kraft entgegen der Stäbchenausrichtung führt zu einer das Stäbchen stauchenden Kraft, die ein Umbiegen des Stäbchens verursacht, in dessen Folge an der fixierenden Oberfläche ein Abrollen stattfindet, das die fixierende Fläche abschält. Dieser Effekt kann auch über mehrere Stäbchenebenen ausgenutzt werden. So kann zum Beispiel nur das untere Ende der Stäbchen mit Vorzugsrichtung ausgestattet sein. Die darauf folgenden zum Beispiel verästelten Strukturen werden abgeschält. Ein gleichwertiger Effekt wird erzielt, wenn die Stäbchen nicht eine Vorzugsbiegerichtung haben, sondern bereits schräg angebracht oder vorgekrümmt sind.

Eine spezielle Ausführung der vorgebogenen oder mit Vorzugsbiegerichtung versehenen Stäbchen ist die, bei der die Stäbchen um einen Drehpunkt, vorzugsweise den Punkt der elektrisch aktiven beziehungsweise sensiblen Fläche in eine Richtung, vorzugsweise entgegen dem Uhrzeigersinn vorgebogen sind. Ein Drehen an der Komponente entgegen dem Uhrzeigersinn rollt jedes einzelne Stäbchenende um die Fixierstelle und schält es ab. So kann die Fixierung durch Andrücken der Komponente oder durch Drehen im Uhrzeigersinn erfolgen. Ein Ablösen erfolgt durch Drehung entgegen dem Uhrzeigersinn.

Neben der genannten tangentialen Ausrichtung der Stäbchen sind weitere Strukturierungen vorstellbar, zum Beispiel eine Fläche, deren Stäbchen in eine Richtung zeigen, ist durch eine Kraft in diese Richtung ablösbar und in die andere Richtung stabil.

Soll die Komponente mit einer orthogonal wirkenden Kraft abgelöst werden, ist es sinnvoll, dass die fixierende Fläche als Membran ausgelegt wird, und die Stäbchen zum Membranmittelpunkt zeigen. Bei senkrechtem Abheben der Komponente lösen sich die Stäbchen von außen nach innen ab. Dieser Vorgang kann alternativ durch einen Stößel, der von innen auf die Membran drückt, oder mit Fluiddruck ausgelöst werden.

Die haftvermittelnde Oberflächenstruktur kann grundsätzlich aus jedem Material gefertigt werden, das sich mit den weiteren Bestandteilen der Elektrodenfixierhülse verbinden lässt und das ausreichend verträglich ist für einen intrakorporalen Einsatz. Die haftvermittelnden Oberflächenstrukturen bestehen bevorzugt aus einem polymeren Werkstoff, insbesondere einem Silikon. Weitere mögliche Werkstoffe für die Strukturen umfassen Kohlenstoffmaterialien, wie Kohlefasern oder Nanotubes, Polypropylene, Polytetrafluorethylen (PTFE), Ethylen-Tetrafluorethylen (ETFE), Polycarbonat, Polystyrol, Polylactide, wie zum Beispiel PDLLA, künstliche Spinnenseide, Polyurethane und Copolymere derselben, Polyimid, Polyamid, Polyetheretherketon (PEEK), Polysulfon, Polyethylen, Polyoxymethylen (POM), Polyetherblockamid, Chitin, Kollagen, Zellulose, Keratin, Metalle, Glas und Keramik verwendet werden.

Eine haftvermittelnde Oberflächenstruktur kann mit unterschiedlichen Verfahren hergestellt werden. Beispielsweise können durch lithographische Verfahren, wie z. B. Elektronenstrahllithographie und Laserlithographie, oder durch Ätzverfahren negative Formen erzeugt werden. In einem anschließenden Gussverfahren wird dann ausgehend von der negativen Form die positive Oberfläche mit haarähnlichen Fortsätzen erzeugt (siehe beispielsweise A.K. Geim et al., Nature Mater. 2, 461-463 (2003) und H. Lee, B.P. Lee and P.B. Messersmith, Nature 448, 338-341 (2007)).

Die Elektrodenfixierhülse umfasst wenigstens einen inneren Hohlkörper und wenigstens einen äußeren Hohlkörper, die miteinander wirkverbunden sind, wobei der innere Hohlkörper mit der haftvermittelnden Oberflächenstruktur versehen ist. Dies bietet insbesondere den Vorteil, dass der innere Hohlkörper mit der haftvermittelnden Oberflächenstruktur in einfacher Weise, zum Beispiel aus einem Gussteil, hergestellt werden kann. Bevorzugt weisen der innere und der äußere Hohlkörper die Form eines Hohlzylinders auf. Weitere Ausführungsformen mit zumindest einer abschnittsweise zylindrischen inneren Mantelfläche sind ebenfalls bevorzugt, wobei diese sich für den Fachmann aus den bekannten geometrischen Formen ableiten lassen. Unter Wirkverbundenheit des inneren und des äußeren Hohlkörpers ist in diesem Zusammenhang gemeint, dass eine auf den inneren oder äußeren Hohlkörper einwirkende Größe, insbesondere eine mechanische Kraft, auf den jeweils anderen Hohlkörper übertragen wird. Vorzugsweise kann eine Übertragungsfunktion gewählt werden, so dass die Übertragung nicht Eins zu Eins erfolgt. Vorzugsweise kann der innere Hohlkörper in einer Formaussparung des äußeren Hohlkörpers angeordnet sein und somit über seine äußeren Oberflächen in dem äußeren Hohlkörper positioniert sein. Die innere Mantelfläche des inneren Hohlkörpers ist mit der haftvermittelnden Oberflächenstruktur versehen.

Der innere und/oder der äußere Hohlkörper weisen elastische Eigenschaften auf. Dies bietet den Vorteil, dass das der Durchmesser der inneren Mantelfläche(n) des inneren und/oder des äußeren Hohlkörper durch elastische Deformation veränderbar sind und die Elektrodenfixierhülse dadurch lösbar befestigt werden kann, ohne dass eine komplexe mechanische Struktur erforderlich ist. Die Erzeugung elastischer Eigenschaften kann auf vielen, dem Fachmann bekannten, Wegen erreicht werden. Bevorzugt erfolgt die Herstellung elastischer Eigenschaften über die Wahl eines elastischen Werkstoffs, zum Beispiel eines Elastomers oder durch die Schaffung elastischer Strukturen, die sich zum Beispiel durch dünne Wandstärken oder große Längen-Breiten-Verhältnisse eines Bauteils realisieren lassen.

Aus dieser Ausgestaltung der Elektrodenfixierhülse geht hervor, dass diese im System einen fixierten und einen nicht-fixierten Zustand haben kann. Der fixierte Zustand beschreibt dabei den Zustand, in dem die haftvermittelnde Oberflächenstruktur einen Kraftfluss durch die Elektrodenleitung und die Elektrodenfixierhülse ermöglicht, mit anderen Worten, die Elektrodenfixierhülse ist auf der Elektrodenleitung befestigt. Der nicht-fixierte Zustand beschreibt das genaue Gegenteil des fixierten Zustands. Im nicht-fixierten Zustand ist also kein Kraftfluss durch die Elektrodenleitung und die Elektrodenfixierhülse möglich, beziehungsweise die Elektrodenfixierhülse ist nicht auf der Elektrodenleitung befestigt. Durch elastische Deformation, beziehungsweise Formation der Elektrodenfixierhülse kann zwischen den Zuständen gewechselt werden. Zu diesem Zweck wird der Elektrodenfixierhülse zum Beispiel eine geeignete Kraft, deren Wirklinie im Wesentlichen radial zur Elektrodenfixierhülse, beziehungsweise orthogonal zur Längsachse der Elektrodenfixierhülse verläuft, aufgeprägt. Wenn von Kraft oder Kräften gesprochen wird, sind damit im Allgemeinen ein oder mehrere Kräftepaare gemeint, deren Wirklinien kollinear sind und deren Wirkrichtung entgegengesetzt ist, wobei die Kräfte den gleichen Betrag haben.

In weiterer bevorzugter Ausgestaltung weist der innere Hohlkörper der Elektrodenfixierhülse wenigstens einen Schlitz auf, der ausgebildet ist, eine elastische Deformation des inneren Hohlkörpers zu ermöglichen und/oder diese zu begrenzen. Dies bietet zum einen den Vorteil, dass der verwendete Werkstoff freier wählbar ist und zum anderen den Vorteil, dass das elastische Verhalten des inneren Hohlkörpers qualitativ und quantitativ ausgelegt werden kann, indem der Schlitz entsprechend gestaltet wird. Der Schlitz muss insbesondere so gestaltet sein, dass durch Begrenzung der elastischen Verformung ein minimaler Durchmesser an der inneren Mantelfläche des inneren Hohlkörpers dem Durchmessers D entspricht. Eine Begrenzung der elastischen Verformung des inneren Hohlkörpers erfolgt vorzugsweise durch Wirkflächenpaarung. Die Wirkflächen können bevorzugt in einem Bereich des Schlitzes angeordnet sein. Besonders bevorzugt können die Wirkflächen ein oder mehrere geometrische Grenzflächen des Schlitzes sein.

In weiterer bevorzugter Ausgestaltung weist die Elektrodenfixierhülse ein Mittel zur Begrenzung der Wirkung einer der Elektrodenfixierhülse aufgeprägten Kraft auf den inneren Hohlkörper auf. Dies bietet den Vorteil, dass der innere Hohlkörper mit der haftvermittelnden Oberflächenstruktur nicht durch eine große angreifende Kraft beschädigt werden kann. Bevorzugt kann das Mittel als ein weiterer Hohlkörper, insbesondere als ein geschlitzter Hohlzylinder vorliegen, der zwischen dem inneren und dem äußeren Hohlkörper angeordnet ist und mit diesen wirkverbunden ist. Vorzugsweise besteht der geschlitzte Hohlzylinder aus einem belastbaren, vorzugsweise metallischen Werkstoff, zum Beispiel Edelstahl. Der Schlitz des Hohlzylinders ermöglicht eine elastische Deformation des weiteren Hohlkörpers, beziehungsweise des geschlitzten Hohlzylinders. Durch die Wirkverbundenheit mit dem inneren und äußeren Hohlkörper, werden also durch eine aufgeprägte Kraft alle drei Hohlkörper elastisch deformiert. Dies führt zu einer Durchmesserverringerung aller drei Hohlkörper. Der Schlitz des weiteren Hohlkörpers, beziehungsweise des geschlitzten Hohlzylinders, muss insbesondere so gestaltet sein, dass durch Begrenzung der elastischen Verformung ein minimaler Durchmesser an der inneren Mantelfläche des inneren Hohlkörpers dem Durchmesser D entspricht. Eine Begrenzung der elastischen Verformung des geschlitzten Hohlzylinders erfolgt vorzugsweise durch Wirkflächenpaarung. Die Wirkflächen können bevorzugt in einem Bereich des Schlitzes angeordnet sein. Besonders bevorzugt können die Wirkflächen ein oder mehrere geometrische Grenzflächen des Schlitzes sein. Der minimale Durchmesser der inneren Mantelfläche des weiteren Hohlkörpers, bevorzugt des geschlitzten Hohlzylinders, korreliert dabei mit dem minimal zugelassenen Durchmesser einer inneren Mantelfläche des inneren Hohlkörpers, der dem Durchmesser D entspricht. Wird die auf die Elektrodenfixierhülse weiter erhöht, erfolgt ein Kraftfluss durch die gepaarten Wirkflächen im Bereich des Schlitzes des geschlitzten Hohlzylinders, so dass keine weitere elastische Verformung mehr auftritt. Die Wirkung der der Elektrodenfixierhülse aufgeprägten Kraft auf den inneren Hohlkörper wird somit vorteilhaft begrenzt und eine Beschädigung in Folge der Krafteinwirkung wird vorteilhaft ausgeschlossen.

In weiterer bevorzugter Ausgestaltung kann das Mittel aus zwei einzelnen identisch geformten Körpern bestehen, die jeweils die Form eines rotationssymmetrischen Teilkörpers aufweisen. Solch einen Körper erhält man, wenn eine rotatorische Formaustragung um weniger als 180° um eine Rotationsachse erfolgt, wobei die rotierende Grundfläche eine Rechteckform aufweist. Liegen zwei solcher Körper vor, kann man diese als zwei unvollständige Hälften eines Hohlzylinders betrachten. Die beiden unvollständigen Hälften können vorzugsweise zwischen dem inneren und dem äußeren Hohlkörper angeordnet werden und mit diesen wirkverbunden sein. Die Rotationsachse der beiden unvollständigen Hälften ist bevorzugt kollinear zur Längsachse des inneren und des äußeren Hohlkörpers angeordnet. Vorzugsweise bestehen die beiden Körper, die unvollständigen Hälften, aus einem belastbaren, vorzugsweise metallischen Werkstoff, zum Beispiel Edelstahl. Eine elastische Deformation des inneren Hohlkörpers in Folge einer der Elektrodenfixierhülse aufgeprägten Kraft ist dann durch Wirkflächenpaarung an den rechteckförmigen Flächen der beiden Körper begrenzt. Die beiden Körper sind vorzugsweise derart ausgelegt, dass eine Unterschreitung des minimalen Durchmessers der inneren Mantelfläche des inneren Hohlkörpers, der dem Durchmesser D entspricht, vermieden wird. Im nicht elastisch deformierten Zustand der Elektrodenfixierhülse korreliert der Abstand der rechteckförmigen Flächen der beiden Körper, die zwischen dem inneren und dem äußeren Hohlkörper angeordnet sind und deren Rotationsachse kollinear zur Längsachse des inneren und des äußeren Hohlkörpers angeordnet ist, also mit der maximal zulässigen elastischen Deformation des inneren Hohlkörpers, beziehungsweise mit dessen maximal zulässiger inneren Durchmesserverringerung.

Das Wirkprinzip des Mittels zur Begrenzung der Wirkung einer der Elektrodenfixierhülse aufgeprägten Kraft auf den inneren Hohlkörper beruht bevorzugt auf dem Prinzip des Formschlusses. Selbiges gilt für die Begrenzung der elastischen Verformung des inneren und des äußeren Hohlkörpers. Somit ist eine Vielzahl weiterer möglicher Ausgestaltungen denkbar, die der Fachmann bei Bedarf vornimmt.

In weiterer bevorzugter Ausgestaltung weist die Elektrodenfixierhülse wenigstens eine umlaufende Nut zur Aufnahme eines Ligaturelements auf. Dies bietet den Vorteil, dass auf Befestigungsmittel wie Ligaturfäden zurückgegriffen werden kann, die im medizinischen Bereich bekannt und meist verfügbar sind. Mit Hilfe solcher Ligaturelemente kann bevorzugt die für die Befestigung, beziehungsweise elastische Deformation, der Elektrodenfixierhülse benötigte Kraft aufgebracht werden. Bevorzugt weist die Elektrodenfixierhülse zwei oder drei umlaufende Nuten zur Aufnahme von Ligaturelementen auf. Weiterhin bevorzugt ist die wenigstens eine umlaufende Nut ausgebildet, um alternative Befestigungselemente aufzunehmen, wie zum Beispiel eine Klemme, einen Clip oder eine Ratsche. Gegebenenfalls ist die Befestigung mit Hilfe der Befestigungselemente reversibel.

Die von dem inneren Hohlkörper bereitgestellte innere Mantelfläche weist in dem fixierten Zustand der Elektrodenfixierhülse im Querschnitt zur Längsachse der Elektrodenfixierhülse eine ovale Kontur, beziehungsweise eine ovale Querschnittsform auf. Der fixierte Zustand liegt bei dieser Ausführungsform dann vor, wenn keine elastische Deformation der Elektrodenfixierhülse vorliegt und diese mit der Elektrodenleitung konzentrisch angeordnet ist. Die haftvermittelnde Oberflächenstruktur ist in wenigstens zwei Teilbereichen der inneren Mantelfläche angeordnet, die sich von zwei Punkten aus erstrecken. Die zwei Punkte sind die im fixierten Zustand im Querschnitt der Elektrodenfixierhülse am dichtesten an der Längsachse der Elektrodenfixierhülse liegenden Punkte der inneren Mantelschicht. Mit anderen Worten: Legt man im fixierten Zustand einen virtuellen Kreis konzentrisch in die Querschnittsform der Elektrodenfixierhülse, so berühren die zwei am dichtesten an der Längsachse der Elektrodenfixierhülse liegenden Punkte der inneren Mantelschicht diesen Kreis tangential, wenn der Kreis den Durchmesser D aufweist oder weiterhin mit anderen Worten, die zwei am dichtesten an der Längsachse der Elektrodenfixierhülse liegenden Punkte der inneren Mantelschicht liegen im fixierten Zustand auf einem virtuellen Kreis, der konzentrisch zur Längsachse der Elektrodenfixierhülse angeordnet ist, wobei eine Ebene, in der der Kreis liegt, normal von der Längsachse der Elektrodenfixierhülse geschnitten wird. Soll die Elektrodenfixierhülse in den nicht-fixierten Zustand gebracht werden, kann dies bevorzugt durch elastische Deformation erfolgen. Hierzu wird zum Beispiel eine Kraft auf die Elektrodenfixierhülse aufgeprägt, deren Wirklinie kollinear zu einer längeren Querachse der ovalen Querschnittsform aufgebracht wird. Bevorzugt weisen sowohl der innere, als auch der äußere Hohlkörper elastische Eigenschaften auf. Durch die aufgeprägte Kraft wird der innere Hohlkörper elastisch deformiert und von einer ovalen Querschnittsform in eine im Wesentlichen kreisförmige Querschnittsform gebracht. Die beiden Punkte entfernen sich dadurch von der Längsachse der Elektrodenfixierhülse. Diese Ausgestaltung der vorliegenden Erfindung bietet den Vorteil, dass nur dann eine Kraft aufgebracht werden muss, wenn die die Elektrodenfixierhülse gelöst werden soll. Die Elektrodenfixierhülse kann somit in einfacher Weise gelöst, auf der Elektrodenleitung verschoben werden und wieder fixiert werden.

Bevorzugt kann die Außenfläche der Elektrodenfixierhülse mit zwei Mulden versehen werden, die entlang der längeren Querachse der ovalen Querschnittsform ausgerichtet sind, so dass die Hülse gelöst wird, wenn sie über diese Mulden zwischen Daumen und Zeigefinger gequetscht wird.

Des Weiteren wird ein Verfahren zur Befestigung einer Elektrodenleitung offenbart, wobei ein Kraftschluss zwischen einer Elektrodenleitung und einem Befestigungselement hergestellt wird. Erfindungsgemäß ist vorgesehen, dass in einem Kontaktbereich des Befestigungselements, zwischen diesem und der Elektrodenleitung, ein definiertes Spaltmaß und/oder eine definierte Anpresskraft erzeugt wird. Erfindungsgemäß werden in dem Kontaktbereich dadurch Oberflächenstrukturen des Befestigungselements so dicht an eine Oberfläche der Elektrodenleitung geführt, dass eine Verbindung entsteht.

Weitere bevorzugte Ausgestaltungen der Erfindung ergeben sich durch beliebige vorteilhafte Kombination der in den Unteransprüchen und der nachfolgenden Beschreibung genannten Merkmale.

Die Erfindung wird nachfolgend anhand eines Ausführungsbeispiels und dazugehöriger Zeichnungen näher erläutert. Die Figuren zeigen:

Fig. 1   Bevorzugtes Ausführungsbeispiel einer erfindungsgemäßen Elektrodenfixierhülse im nicht-fixierten Zustand als Explosionsdarstellung (ohne Elektrodenleitung)

Fig. 2   Bevorzugtes Ausführungsbeispiel einer erfindungsgemäßen Elektrodenfixierhülse im fixierten Zustand als Schnittansicht und Ansicht entlang einer Längsachse (ohne Elektrodenleitung)

Fig. 3A/B   Alternative Ausführungsformen eines inneren Hohlkörpers

Fig. 4   Alternative Ausführungsvarianten eines Mittels zur Begrenzung der Wirkung, einer der Elektrodenfixierhülse aufgeprägten Kraft, auf einen inneren Hohlkörper

Fig. 5   Querschnittsansicht einer erfindungsgemäßen Elektrodenfixierhülse im fixierten Zustand mit einer ovalen Querschnittsform einer inneren Mantelfläche des inneren Hohlkörpers in einer alternativen Ausführungsform

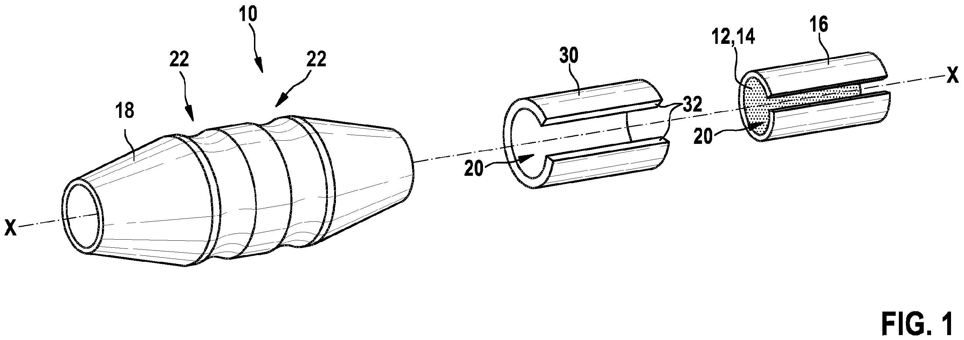

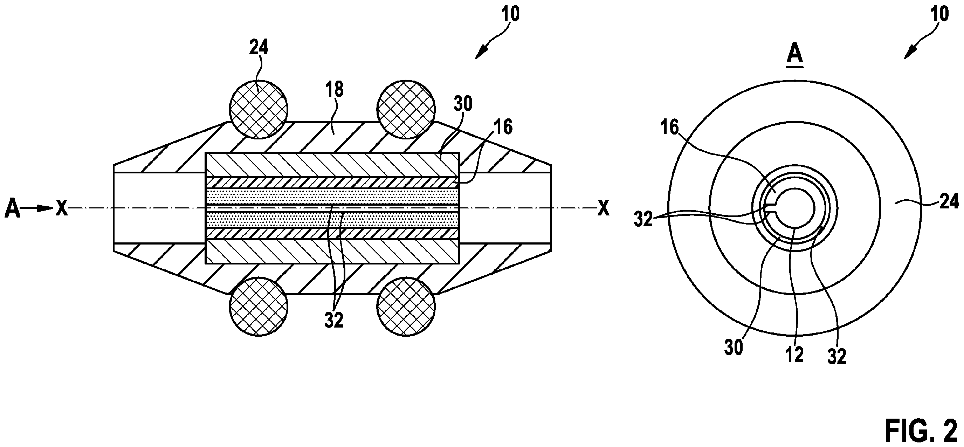

Figuren 1 und 2 zeigen ein bevorzugtes Ausführungsbeispiel einer erfindungsgemäßen Elektrodenfixierhülse 10 in einem nicht-fixierten Zustand als Explosionsdarstellung sowie als Schnittansicht und in einer Ansicht entlang einer Längsachse X der Elektrodenfixierhülse 10. Die Elektrodenleitung ist nicht mit dargestellt.

Die Elektrodenfixierhülse 10 weist erfindungsgemäß eine innere Mantelfläche 12 auf, die mit einer haftvermittelnden Oberflächenstruktur 14 versehen ist. Weiterhin weist die Elektrodenfixierhülse 10 zwei umlaufende Nuten 22 zur Aufnahme von Ligaturelementen auf. Die haftvermittelnde Oberflächenstruktur 14 ist als Geckostruktur ausgebildet. Die Elektrodenfixierhülse 10 umfasst einen inneren Hohlkörper 16 und einen äußeren Hohlkörper 18 sowie Mittel zur Begrenzung der Wirkung einer der Elektrodenfixierhülse 10 aufgeprägten Kraft auf den inneren Hohlkörper 16, die miteinander wirkverbunden sind. Der innere Hohlkörper 16 ist mit der haftvermittelnden Oberflächenstruktur 14 versehen.

Das Mittel zur Begrenzung der Wirkung einer der Elektrodenfixierhülse 10 aufgeprägten Kraft auf den inneren Hohlkörper 16 ist als ein Hohlzylinder 30 mit einem entlang der Längsachse X durchgehend ausgebildeten Schlitz 20 ausgebildet. Der geschlitzte Hohlzylinder 30 ist zwischen dem inneren Hohlkörper 16 und dem äußeren 18 Hohlkörper angeordnet und mit diesen wirkverbunden. Der geschlitzte Hohlzylinder 30 besteht aus Edelstahl. Der innere Hohlkörper 16 besteht aus Polyamid und der äußere Hohlkörper 18 besteht aus einem Silikon. Der innere Hohlkörper 16 weist einen entlang der Längsachse X durchgehend ausgebildeten Schlitz 20 auf. Die Schlitze 20 des Hohlzylinders 30 und des inneren Hohlkörpers 16 sowie elastische Werkstoffeigenschaften des äußeren Hohlkörpers 18 ermöglichen eine elastische Deformation der Elektrodenfixierhülse 10. Durch die Wirkverbundenheit des geschlitzten Hohlzylinders 30 mit dem inneren Hohlkörper 16 und dem äußeren Hohlkörper 18 können durch eine aufgeprägte Kraft alle drei Körper elastisch deformiert werden. Der Schlitz 20 des Hohlzylinders 30 ist so ausgebildet, dass bei Erreichen eines minimalen Durchmessers D an der inneren Mantelfläche 12 des inneren Hohlkörpers 16 eine Begrenzung der elastischen Verformung des geschlitzten Hohlzylinders 30 durch Paarung der Wirkflächen 32 erfolgt. Da auch bei einer weiteren Erhöhung der auf die Elektrodenfixierhülse 10 wirkenden Kraft keine weitere elastische Verformung möglich ist, verringert sich auch der Durchmesser D nicht weiter. Im fixierten Zustand stellt sich im System mit einer Elektrodenleitung ein Spaltmaß R, zwischen der inneren Mantelfläche 12 des inneren Hohlkörpers 16 und der Oberfläche der Elektrodenleitung ein. Die Wirkflächen 32 des inneren Hohlkörpers 16 berühren sich dabei nicht.

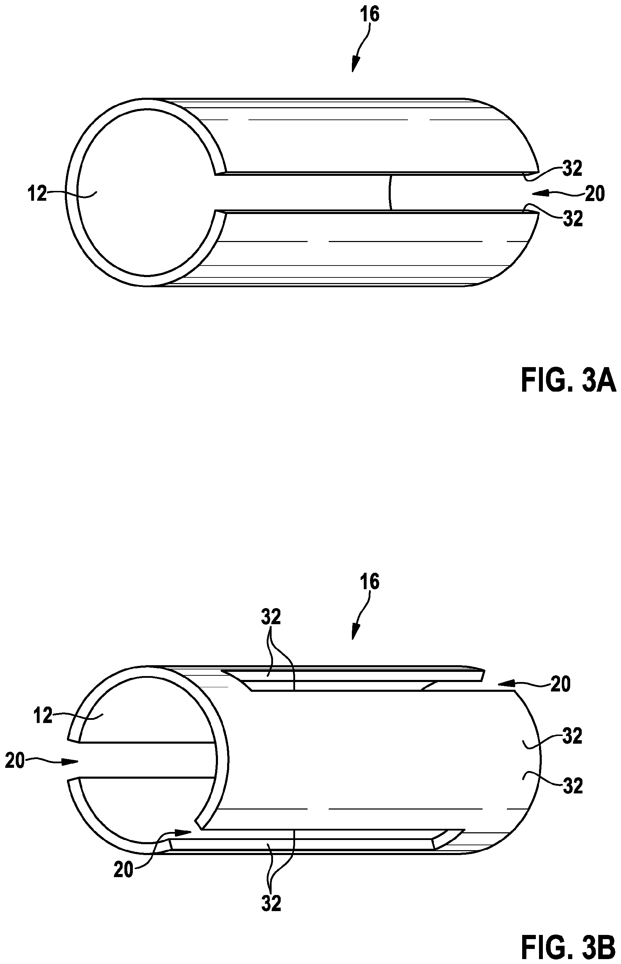

Den Figuren 3A und 3B sind zwei alternative Ausführungsformen des inneren Hohlkörpers zu entnehmen. Die Figur 3A zeigt einen inneren Hohlkörper 16 mit innerer Mantelfläche 12 und Wirkflächen 32 in einem Bereich des Schlitzes 20. Der Schlitz ist durchgängig über die volle Länge des inneren Hohlkörpers 16 ausgebildet. Diese Variante bietet den Vorteil, dass die elastische Verformung gleichmäßig erfolgt und dass der innere Hohlkörper 16 mit diesem Schlitz 20 einfach hergestellt werden kann.

Die Figur 3B zeigt eine weitere Ausführungsform des inneren Hohlkörpers 16 mit mehreren Schlitzen. Der innere Hohlkörper 16 weist in dieser Variante drei Schlitze 20 auf, die nicht durchgängig über die volle Länge des inneren Hohlkörpers 16 ausgebildet sind. Je nachdem, über welchen Anteil der Länge des inneren Hohlkörpers 16 sich die Schlitze 20 erstrecken, nehmen die elastischen Eigenschaften des inneren Hohlkörpers 16 zu oder ab. Dies bietet den Vorteil, dass die elastischen Eigenschaften genau einstellbar sind.

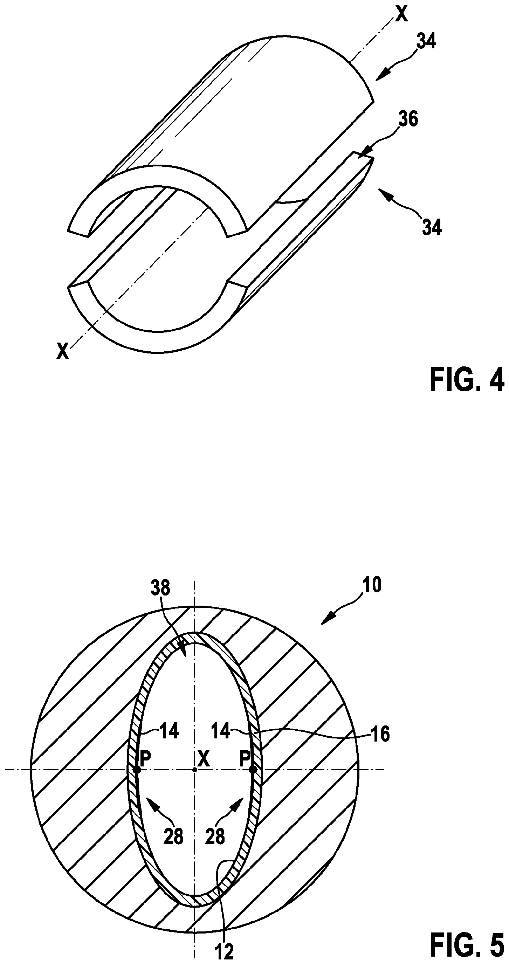

Figur 4 zeigt eine alternative Ausführungsvariante eines Mittels zur Begrenzung der auf den inneren Hohlkörper 16 wirkenden Kraft, die durch Aufprägen einer Kraft auf den äußeren Hohlkörper 18 der Elektrodenfixierhülse 10 entsteht. Das Mittel besteht aus zwei einzelnen, identisch geformten Körpern 34, die jeweils die Form eines rotationssymmetrischen Teilkörpers aufweisen. Die Körper 34 lassen sich abstrakt als das Produkt einer rotatorischen Formaustragung um weniger als 180° um eine Rotationsachse, die gleichzeitig eine Längsachse X der Körper 34 bildet, beschreiben. Die der rotatorischen Formaustragung zugrunde liegende Rotations- oder Grundfläche 36 weist eine Rechteckform auf. Die zwei Körper 34 stellen zwei unvollständige Hälften eines Hohlzylinders dar. Die beiden Körper 34 bestehen aus Edelstahl. Jeder der Körper 34 weist zwei Wirkflächen 32 auf, die der Grundfläche 36 der Rotationsaustragung entsprechen. Geometrische Abwandlungen der Wirkflächen 32 können zu vorteilhafteren Eigenschaften im Falle einer Wirkflächenpaarung vorgenommen werden.

Figur 5 zeigt eine Querschnittsansicht einer erfindungsgemäßen Elektrodenfixierhülse 10 in einem fixierten Zustand mit einer ovalen Querschnittsform 38 einer inneren Mantelfläche 12 des inneren Hohlkörpers 16 in einer alternativen Ausführungsform. Die von dem inneren Hohlkörper 16 bereitgestellte innere Mantelfläche 12 weist in dem fixierten Zustand der Elektrodenfixierhülse 10 im Querschnitt zur Längsachse X der Elektrodenfixierhülse 10 eine ovale Kontur, beziehungsweise eine ovale Querschnittsform 38 auf. Der fixierte Zustand liegt bei dieser Ausführungsform dann vor, wenn keine elastische Deformation der Elektrodenfixierhülse 10 vorliegt. Die haftvermittelnde Oberflächenstruktur 14 ist in wenigstens zwei Teilbereichen 28 der inneren Mantelfläche 12 angeordnet, die sich von zwei Punkten P aus über einen Teil der inneren Mantelfläche 12 erstrecken. Die zwei Punkte P sind die im fixierten Zustand im Querschnitt der Elektrodenfixierhülse 10 am dichtesten an der Längsachse X der Elektrodenfixierhülse liegenden Punkte der inneren Mantelfläche 12.

## Patentansprüche

**1.** Elektrodenfixierhülse (10), bei der eine innere Mantelfläche (12) der Elektrodenfixierhülse (10) zumindest teilweise mit einer haftvermittelnden Oberflächenstruktur (14) versehen ist, wobei die Elektrodenfixierhülse (10) wenigstens einen inneren Hohlkörper (16) und wenigstens einen äußeren Hohlkörper (18) umfasst, die miteinander wirkverbunden sind, wobei der innere (16) und/oder der äußere Hohlkörper (18) elastische Eigenschaften aufweist, wobei der innere Hohlkörper (16) mit der haftvermittelnden Oberflächenstruktur (14) versehen ist, **dadurch gekennzeichnet, dass** die von dem inneren Hohlkörper (16) bereitgestellte innere Mantelfläche (12) in einem fixierten Zustand der Elektrodenfixierhülse (10) im Querschnitt zur Längsachse (X) der Elektrodenfixierhülse (10) eine ovale Kontur (26) aufweist, wobei die haftvermittelnde Oberflächenstruktur (14) in wenigstens zwei Teilbereichen (28) der Mantelfläche (12) angeordnet ist, die sich, ausgehend von zwei am dichtesten an der Längsachse der Elektrodenfixierhülse (10) liegenden Punkten (P) der inneren Mantelfläche (12), erstrecken.

**2.** Elektrodenfixierhülse (10) nach Anspruch 1, bei der der innere Hohlkörper (16) wenigstens einen Schlitz (20) aufweist, der ausgebildet ist, eine elastische Deformation des inneren Hohlkörpers (16) zu ermöglichen und/oder diese zu begrenzen.

**3.** Elektrodenfixierhülse (10) nach Anspruch 1 oder 2, bei der die Elektrodenfixierhülse (10) ein Mittel zur Begrenzung der Wirkung einer der Elektrodenfixierhülse (10) aufgeprägten Kraft auf den inneren Hohlkörper (16) aufweist.

**4.** Elektrodenfixierhülse (10) nach einem der vorangegangenen Ansprüche, bei der die Elektrodenfixierhülse (10) wenigstens eine umlaufende Nut (22) zur Aufnahme eines Ligaturelements (24) aufweist.

**5.** Elektrodenfixierhülse (10) nach einem der Ansprüche 1 bis 4, bei der der innere Hohlkörper (16) und/oder der äußere Hohlkörper (18) und/oder die haftvermittelnde Oberflächenstruktur (14) aus einem polymeren Werkstoff bestehen.

**6.** System, bestehend aus einer Elektrodenfixierhülse (10) nach einem der Ansprüche 1 bis 5 und einer Elektrodenleitung, wobei die Elektrodenfixierhülse (10) konzentrisch mit der Elektrodenleitung angeordnet ist und sich dabei ein radiales Spaltmaß R zwischen einer inneren Mantelfläche (12) der Elektrodenfixierhülse (10) und einer äußeren Oberfläche der Elektrodenleitung einstellt, das zwischen 10 $\mu$m und 1500 $\mu$m, weiterhin bevorzugt zwischen 20 $\mu$m und 750 $\mu$m und besonders bevorzugt zwischen 50 $\mu$m und 300$\mu$m beträgt.

## Claims

**1.** An electrode fixing sleeve (10), in which an inner lateral surface (12) of the electrode fixing sleeve (10) is provided at least partially with an adhesion-promoting surface structure (14), wherein the electrode fixing sleeve (10) comprises at least one inner hollow body (16) and at least one outer hollow body (18), which are operatively connected to one another, wherein the inner hollow body (16) and/or the outer hollow body (18) have/has resilient properties, wherein the inner hollow body (16) is provided with the adhesion-promoting surface structure (14), **characterised in that** the inner lateral surface (12) provided by the inner hollow body (16) in a fixed state of the electrode fixing sleeve (10) has an oval contour (26) in a cross section relative to the longitudinal axis (X) of the electrode fixing sleeve (10),

wherein the adhesion-promoting surface structure (14) is arranged in at least two sub-regions (28) of the lateral surface (12), which extend starting from two points (P) of the inner lateral surface (12) lying closest to the longitudinal axis of the electrode fixing sleeve (10).

2. The electrode fixing sleeve (10) according to claim 1, in which the inner hollow body (16) has at least one slot (20), which is designed to enable and/or limit an elastic deformation of the inner hollow body (16).

3. The electrode fixing sleeve (10) according to claim 1 or 2, in which the electrode fixing sleeve (10) comprises a means for limiting the effect of a force exerted on the electrode fixing sleeve (10) on the inner hollow body (16).

4. The electrode fixing sleeve (10) according to any one of the preceding claims, in which the electrode fixing sleeve (10) comprises at least one peripheral groove (22) for receiving a ligature element (24).

5. The electrode fixing sleeve (10) according to any one of claims 1 to 4, in which the inner hollow body (16) and/or the outer hollow body (18) and/or the adhesion-promoting surface structure (14) consist/consists of a polymer material.

6. A system, consisting of an electrode fixing sleeve (10) according to any one of claims 1 to 5 and an electrode lead, wherein the electrode fixing sleeve (10) is arranged concentrically with the electrode lead and a radial gap R is provided between an inner lateral surface (12) of the electrode fixing sleeve (10) and an outer surface of the electrode lead, said gap being between 10 $\mu$m and 1500 $\mu$m, more preferably between 20 $\mu$m and 750 $\mu$m, and particularly preferably between 50 $\mu$m and 300 $\mu$m.

**Revendications**

1. Manchon de fixation d'électrode (10) chez lequel une surface d'enveloppe intérieure (12) du manchon de fixation d'électrode (10) est muni au moins partiellement d'une structure de surface (14) favorisant l'adhérence,

où le manchon de fixation d'électrode (10) comprend au moins un corps creux intérieur (16) et au moins un corps creux extérieur (18), qui sont reliés de manière fonctionnelle,

où le corps creux intérieur (16 et/ou extérieur (18) présente des propriétés élastiques, où le corps creux intérieur (16) est muni de la structure de surface (14) favorisant l'adhérence, **caractérisé en ce que** la surface d'enveloppe intérieure (12) procurée par le corps creux intérieur (16) présente, dans un état fixé du manchon de fixation d'électrode (10) un contour (26) ovale dans la section transversale par rapport à l'axe longitudinal (X) du manchon de fixation d'électrode (10),

où la structure de surface (14) favorisant l'adhérence est disposée dans au moins deux zones partielles (28) de la surface d'enveloppe (12) qui s'étendent en partant de deux points (P) de la surface d'enveloppe intérieure (12) situés le plus près de l'axe longitudinal du manchon de fixation d'électrode (10).

2. Manchon de fixation d'électrode (10) selon la revendication 1, chez lequel le corps creux intérieur (16) présente au moins une fente (20) qui est prévue pour permettre une déformation élastique du corps creux intérieur (16) et/ou pour limiter celle-ci.

3. Manchon de fixation d'électrode (10) selon la revendication 1 ou 2, chez lequel le manchon de fixation d'électrode (10) présente un moyen destiné à la limitation de l'action d'une force mise en oeuvre par le manchon de fixation d'électrode (10) sur le corps creux intérieur (16).

4. Manchon de fixation d'électrode (10) selon l'une des revendications précédentes, chez lequel le manchon de fixation d'électrode (10) présente une rainure circonférentielle (22) pour l'admission d'un élément de ligature (24).

5. Manchon de fixation d'électrode (10) selon l'une des revendications 1 à 4, chez lequel le corps creux intérieur (16) et/ou le corps creux extérieur (18) et/ou la structure de surface (14) favorisant l'adhérence sont constitués d'un matériau polymère.

6. Système, constitué d'un manchon de fixation d'électrode (10) selon l'une des revendications 1 à 5 et d'une ligne d'électrode, où le manchon de fixation d'électrode (10) est disposé concentriquement avec la ligne d'électrode et qu'il se forme une fente radiale R entre une surface d'enveloppe intérieure (12) du manchon de fixation d'électrode (10) et une surface extérieure de la ligne d'électrode, qui se situe entre 10 $\mu$M et 1500 $\mu$m, en outre, de préférence entre 20 $\mu$m et 750 $\mu$m et de manière particulièrement préférée entre 50 $\mu$m et 300 $\mu$m.

**FIG. 1**

FIG. 2

EP 3 106 201 B1

16

32
20
32

12

**FIG. 3A**

16
32
12
20
20
32
32
20
32

**FIG. 3B**

**FIG. 4**

**FIG. 5**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 8224292 U1

- DE 102005015487 B3

**In der Beschreibung aufgeführte Nicht-Patentliteratur**

- **ALBORZ MAHDAVI et al.** A biodegradable and biocompatible gecko-inspired tissue adhesive. *PNAS,* 2008, vol. 105 (7), 2307-231

- **A.K. GEIM et al.** *Nature Mater.,* 2003, vol. 2, 461-463
- **H. LEE ; B.P. LEE ; P.B. MESSERSMITH.** *Nature,* 2007, vol. 448, 338-341